# EUROPEAN PATENT SPECIFICATION

(11) **EP 1 172 108 B1**
(45) Date of publication and mention of the grant of the patent: **22.12.2004**
(21) Application number: 00906630.9
(22) Date of filing: 02.03.2000
(51) Int. Cl.: A61K 31/7032, A61P 37/06

(54) **Use of Sulfoquinovosyldiacylglycerol derivatives as immunosuppressants**
Verwendung von Sulfoquinovosyldiacylglycerol Derivativen als Immunosuppresssiva
Utilisation de Dérivés de Sulfoquinovosyldiacylglycérol comme Immunosuppresseurs

(30) Priority: 11.03.1999 JP 6520899; 23.06.1999 JP 17688199
(43) Date of publication of application: 16.01.2002
(73) Proprietor: Toyo Suisan Kaisha, Ltd., Tokyo 108-8501 (JP)
(72) Inventor: Yamazaki, Takayuki, Noda-shi, Chiba 278-0022 (JP); Sugawara, Fumio, Niiza-shi, Saitama 352-0012 (JP); Ohta, Keisuke, Noda-shi, Chiba 278-0022 (JP); Masaki, Kazuyoshi, Sakado-shi, Saitama 350-0203 (JP); Nakayama, Kotaro, Yotsukaido-shi, Chiba 284-0025 (JP); Sakaguchi, Kengo, Tsukuba-shi, Ibaraki 300-2667 (JP); Sato, Noriyuki, Sapporo-shi, Hokkaido 062-0042 (JP); SAHARA, Hiroeki, Sapporo-shi, Hokkaido 005-0855 (JP); FUJITA, Tatsuya, Minami-ku, Sapporo-shi (JP)
(74) Representative: Chapman, Paul William
(86) International application number: PCT/JP2000/001231
(87) International publication number: WO 2000/053190

(56) References cited:
- FR-A- 2 774 094
- JP-A- 3 052 815
- JP-A- 3 052 816
- JP-A- 7 242 691
- JP-A- 11 106 395
- OHTA K ET AL: "Sulfoquinovosyldiacylglycerol, KM043, a new potent inhibitor of eukaryotic DNA polymerases and HIV-reverse transcriptase type 1 from a marine red alga, Gigartina tenella" CHEMICAL AND PHARMACEUTICAL BULLETIN, PHARMACEUTICAL SOCIETY OF JAPAN. TOKYO, JP, vol. 46, no. 4, 1998, pages 684-686, XP002136650 ISSN: 0009-2363
- JERZY GOLIK, ET. AL.: 'Isolation and Structure Determination of Sulfoquinovosyl Dipalmitoyl Glyceride, a P-Selectin Receptor Inhibitor from the Alga Dictyochloris fragrans' J. NAT. PROD. vol. 60, no. 4, 1997, pages 387 - 389, XP002928047
- BANNIKUPPE A. VISHWANATH ET. AL.: 'Interaction of plant lipids with 14kDa phospholipase A2 enzymes.' BIOCHEM. J. vol. 320, no. 1, 1996, pages 93 - 99, XP002928048

## Description

### Technical Field

The present invention relates to the use of sulfoquinovosyldiacylglycerol derivatives as immunosuppressive agents.

### Background Art

In clinical treatment presently performed, transplantation can be employed to treat chemotherapeutically untreatable diseases. Transplantation is the technology for treating a disease by replacing partly or entirely of a diseased organ with a healthy organ taken from another individual. Organ transplantation has been performed with respect to a wide variety of organs such as kidney, liver, lung, intestine, heart, pancreas, and cornea. The number of organ transplantations has been increased.

The immune response of skin is inherently high. However, skin transplantation can be made successfully if a graft skin transplanted from one person to another can be kept alive for at least a few weeks. This is because new dermal tissue, if a graft epidermis is kept alive for a few weeks, can regenerate itself, thereby recovering from a dermal tissue damage. Therefore, it is possible to make physical recuperation of serious and extensive burn or laceration by transplanting a dermal tissue from another person.

The most fearful problem residing in tissue or organ transplantation is a rejection caused by a recipient's immune response.

Under these circumstances, in order to develop an immunosuppressive agent capable of preventing the rejection in a recipient, thereby attaining permanent fixation of a transplanted organ, intensive studies have been conducted since the 1970s, particularly in European countries and U.S.A.

On the other hand, an immunosuppressive agent may also be important in treating autoimmune diseases such as rheumatism and collagen disease, since it can mitigate the symptoms to a certain degree.

Up to the present, cyclosporin A and FK506, etc., have been developed as immunosuppressive agents. However, the functional mechanisms of these immunosuppressive agents resemble each other and their chronic toxicity is a matter of concern. Thus, to attain prolonged life in next-generation organ transplantation, another type of immunosuppressive agent is desired which has a lower toxicity based on a different chemical structure, and thus, different functional mechanism can be expected.

It has been found that naturally-occurring sulfur-containing glycolipids have pharmaceutical activities such as an anticancer effect (Sahara et al., British Journal of Cancer, 75(3), 324-332, (1997)); inhibitory activities against DNA polymerase (Mizushina et al., Biochemical Pharmacology, 55, 537-541 (1998), Ohta et al., Chemical & Pharmaceutical Bulletin, 46(4), 1998)); and HIV suppressive effect (National Patent Publication No. 5-501105). However, it has not yet been found that a sulfur-containing glycolipid, in particular, a sulfoquinovosyldiacylglycerol derivative, has an immunosuppressive activity.

### Disclosure of Invention

An object of the present invention is to provide sulfoquinovosyldiacylglycerol derivatives as immunosuppressive agents. More specifically, the object of the present invention is to provide an immunosuppressive agent showing low toxicity and usability of long-term administration, and high immunosuppressive activity as well.

The present inventors have conducted studies to attain the aforementioned object. As a result, they found that specific sulfoquinovosyldiacylglycerol derivatives have a remarkable immunosuppressive activity and accomplished the present invention. The present invention provides an immunosuppressive agent containing, as an active ingredient, at least one compound selected from
compounds represented by Formula (1): 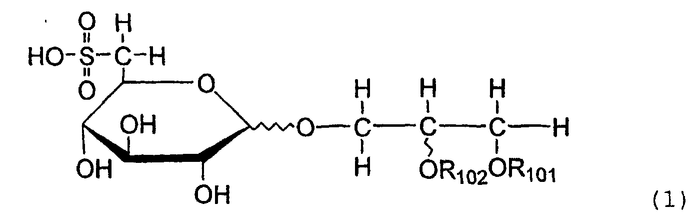 where R₁₀₁ and R₁₀₂ independently represent an acyl residue of a higher fatty acid, represented by R-C(=O), wherein R represents an alkyl or alkenyl group having 13 or more carbon atoms and
a pharmaceutically acceptable salt thereof.

### Brief Description of Drawings

FIG. 1 shows the relationship between the concentration of the compound (SQAG 3) represented by Formula (1) and the immunosuppressive activity;
FIG. 2 shows the relationship between the concentration of the compound (SQAG 5) represented by Formula (1) and the immunosuppressive activity;
FIG. 3 shows the relationship between the concentration of the compound (SQAG 7) represented by Formula (1) and the immunosuppressive activity; and
FIG. 4 shows the relationship between the concentration of the compound (SQAG 9) represented by Formula (1) and the immunosuppressive activity.

### Best Mode for Carrying Out of the Invention

In the specification, the term "carbon atoms" of a protecting group refers to the number of carbon atoms assuming that the protecting group is unsubstituted.

In the first place, we will more specifically explain the active ingredient contained in the immunosuppressive agent of the present invention, that is, a sulfoquinovosyldiacylglycerol derivative represented by Formula (1): 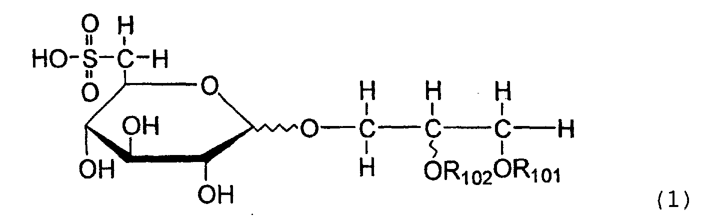 where R₁₀₁ and R₁₀₂ independently represent an acyl residue of a higher fatty acid.

In Formula (1), R₁₀₁ represents an acyl residue of a higher fatty acid. Fatty acids giving the acyl residue represented by R₁₀₁ include straight-chain or branched-chain, saturated or unsaturated higher fatty acids.

The acyl residues of straight-chain or branched-chain higher fatty acids represented by R₁₀₁ include groups represented by R-C(=O), where R represents an alkyl or alkenyl group having 13 or more carbon atoms. The number of carbon atoms of the alkyl and alkenyl groups represented by R of R-C(=O) is preferably 13 or more and 25 or less, and more preferably, an odd number within 15-25. This is because if the number of carbon atoms of R exceeds 25, the manufacturing cost increases.

In Formula (1), R₁₀₂ has the same meaning as R₁₀₁.

R₁₀₁ and R₁₀₂ may be the same or different. However, they are preferably the same in view of manufacturing facility.

The sugar skeleton of sulfoquinovoside in Formula (1) may take either a boat or chair conformation. However, the chair conformation is preferable in view of stability. The absolute configuration of the carbon (asymmetric carbon) at the 2-position of the glycerol moiety may be either the S-or R-configuration.

The bonding between sulfoquinovoside and glycerol is either an α- or β-bonding. However, the β-bonding is preferable judging from the results of the immunosuppressive activity assay using cultured cells.

The sulfoquinovosyldiacylglycerol derivatives of the present invention can be prepared via (Step A) to (Step J) in accordance with the following reaction procedure.

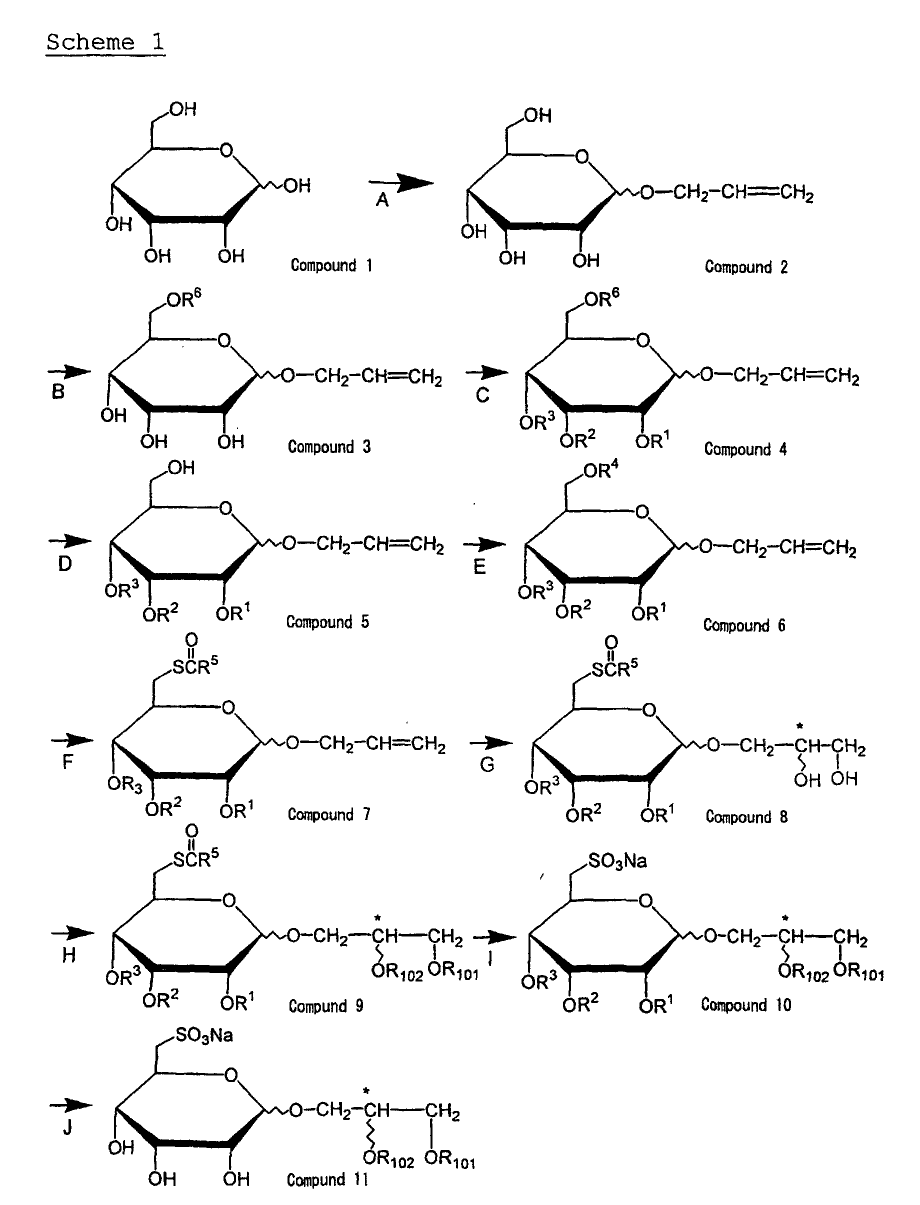

(Step A) The hydroxyl group bonded to the C1 carbon of D-glucose is converted into a 2-propenyl group. (Step B) The hydroxyl group of the C6 carbon of the glucose is protected. (Step C) The hydroxyl groups bonded to the C2, C3 and C4 carbons of the glucose are protected. (Step D) The protecting group of the C6 carbon previously protected is deprotected. (Step E) The hydroxyl group bonded to the C6 carbon is substituted with a group (for example, an alkylsulfonyloxy group or arylsulfonyloxy group) which can be converted to a carbonylthio group. (Step F) The C6 carbon is converted into a carbonylthio group. (Step G) The 2-propenyl group bonded to the C1 carbon is converted into a diol. (Step H) Each of the hydroxyl groups of the diol thus obtained is esterified with a desired higher fatty acid. (Step I) The carbonylthio group at the C6 carbon is converted into a sulfonate salt. (Step J) The protecting groups of C2, C3 and C4 carbons of the sulfonate salt obtained are deprotected. As a result, a salt of a sulfoquinovosyldiacylglycerol derivative of the present invention can be produced. The salt thus obtained is subjected to titration with an acid such as hydrochloric acid to give the sulfoquinovosyldiacylglycerol derivative of the present invention.

The aforementioned Steps A-J will be further explained in detail.

In Step A, the 2-propenylation is carried out by reacting the glucose with allyl alcohol in the presence of a strong acid, such as trifluoromethanesulfonic acid, usually at room temperature to 100°C, preferably from 80 to 90°C, for a half day to two days. However, the reaction time varies depending upon the reaction conditions.

In Step B, the hydroxyl group bonded to the C6 carbon is protected to obtain the compound to which -OR⁶ is bonded at the C6 carbon (where R⁶ represents an alkyl or substituted silyl group).

As the compound capable of protecting the hydroxyl group, a compound can be used which can provide an alkyl or substituted silyl group as the R⁶ group.

Examples of the alkyl group represented by R⁶ preferably include bulky and substituted lower alkyl groups. The substituents of the bulky and substituted alkyl groups include methyl and phenyl groups. The specific examples of the substituted alkyl group include t-butyl and trityl groups.

When the group represented by R⁶ represents a substituted silyl group, examples of substituents of the substituted silyl group include lower alkyl groups, preferably alkyl groups having 1-4 carbon atoms (for example, methyl, ethyl, isopropyl and t-butyl groups); and aryl groups, preferably aryl groups having 6 carbon atoms (for example, a phenyl group). The substituted silyl group represented by R⁶ preferably includes trisubstituted silyl groups, more preferably, a t-butyldiphenylsilyl group.

When the compound 3, where R⁶ represents an alkyl group, is to be obtained, the protection of the hydroxyl group in Step B can be carried out by adding a compound represented by R⁶-X (where R⁶ represents the alkyl group defined above, and X represents a halogen atom such as chlorine atom) to a solution of the compound 2 dissolved in an organic solvent, such as anhydrous pyridine, and reacting the solution mixture at room temperature in the presence of a catalyst such as p-dimethylaminopyridine (DMAP). As the compound R⁶-X, trityl chloride is preferably used in view of manufacturing cost and reaction facility.

When the compound 3, where R⁶ represents a substituted silyl group, is to be obtained, t-butyldiphenylsilyl chloride, for example, is used as the compound R⁶-X, and the reaction is carried out in the presence of a catalyst, such as imidazole, at room temperature for a half day to two days. Note that the reaction time varies depending upon the reaction conditions.

In Step C, the hydroxyl groups bonded to the C2, C3 and C4 carbons are protected and converted into -OR¹, -OR² and -OR³, respectively, where R¹ to R³ independently represent an alkyl or substituted silyl group. The protection of these hydroxyl groups can be carried out by activating, with sodium hydride, the hydroxyl groups bonded to the C2, C3 and C4 carbons of the compound 3 dissolved in an organic solvent, such as N, N-dimethylformamide (DMF), and reacting with the compound capable of protecting these hydroxyl groups at room temperature.

As the compound capable of protecting the hydroxyl groups, benzyl bromide, p-methoxybenzyl bromide, t-butyldimethylsilyl chloride or triethylsilyl chloride may be used. The reaction using the compound capable of protecting the hydroxyl groups can be carried out under a suitable reaction condition for each of the protecting groups.

The deprotection of the protecting group bonded to the C6 carbon in Step D may be carried out by reacting a solution of the compound 4 dissolved in an organic solvent, such as methanol, in the presence of a catalyst, such as p-toluenesulfonic acid, for a half day to one day at room temperature. The reaction time varies depending upon the reaction conditions.

In Step E, R⁴, that is, an alkylsulfonyl or arylsulfonyl group is bonded to the hydroxyl group at the C6 carbon of the compound 5, so that the hydroxyl group is converted into -OR⁴ to give the compound 6.

The reaction to give the -OR⁴ group is performed by adding a compound having the alkylsulfonyl group or a compound having the arylsulfonyl group to a solution of the compound 5 dissolved in an organic solvent, and reacting them. The alkyl groups of the compound having the alkylsulfonyl group preferably include unsubstituted alkyl groups, more preferably, lower alkyl groups, much more preferably, alkyl groups having 1-2 carbon atoms (methyl and ethyl groups). The compound having an alkylsulfonyl group can be represented by R⁴'-X, where R⁴' represents an alkylsulfonyl group, and X represents a halogen atom. Specific examples include methanesulfonyl chloride and ethanesulfonyl chloride.

On the other hand, the aryl group of the compound having the arylsulfonyl group may include unsubstituted and substituted aryl groups, preferably aryl groups having 6 carbon atoms (e.g., a phenyl group). In the case of the substituted aryl group, examples of the substituent thereof include p-methyl and p-methoxy groups. Examples of the compound having an arylsulfonyl group include compounds represented by R⁴"-X, where R⁴" represents an arylsulfonyl group, and X represents a halogen atom. Specific examples include p-toluenesulfonyl chloride, p-methoxybenzenesulfonyl chloride and benzenesulfonyl chloride.

Of the compounds having an alkylsulfonyl or arylsulfonyl group, a compound having a tosyl group is preferably used from the viewpoint of reaction facility.

In the reaction of Step E, as an organic solvent, for example, pyridine or dichloromethane may be used.

The reaction mentioned above may be performed, as the case may be, in the presence of a catalyst, such as DMAP, at room temperature for 2 hours to one day. The reaction time varies depending upon the reaction conditions.

In Step F, the sulfonyloxy group (-OR⁴) of the compound 6 is replaced with a carbonylthio group represented by -SC (=O)R⁵, where R⁵ represents a hydrogen atom, an alkyl or aryl group.

In the reaction, a compound capable of substituting the alkylsulfonyloxy or arylsulfonyloxy group of the compound 6 with the carbonylthio group, is allowed to react in an organic solvent to give a compound 7. Hereinafter, this compound will be referred to as "O-substituted → S-substituted compound".

Examples of the O-substituted → S-substituted compound include alkali metal salts and alkali earth metal salts of a thiocarboxylic acid. Examples of the thiocarboxylic acid include thioformic acid, lower thiocarboxylic acids, preferably aliphatic thiocarboxylic acids each having 1-5 carbon atoms in its aliphatic hydrocarbon moiety (for example, thioacetic acid or thiopropionic acid), and aromatic thiocarboxylic acids each having 6-10 carbon atoms in its aromatic hydrocarbon moiety (for example, thiobenzoic acid).

The alkali metal that forms a salt with the thiocarboxylic acid includes potassium and sodium. The alkali earth metal includes magnesium and calcium.

Of the above-mentioned O-substituted → S-substituted compounds, salts of thioacetic acid may be preferably used since a reaction can proceed stably and the sulfur atom can be easily oxidized in a later step.

Examples of an organic solvent used in the reaction include alcohol, for example, methanol, ethanol and propanol, N,N-dimethylformamide and dimethylsulfoxide.

The aforementioned reaction may be performed usually at room temperature to the boiling point of a solvent to be used while stirring for one hour to one day. Note that the reaction time varies depending upon the reaction conditions.

The dihydroxylation of Step G may be performed by adding an oxidizing agent, such as osmium tetraoxide, to a solution of the compound 7 dissolved in a solvent mixture, such as a mixture of t-butanol and water, and then reacting the resultant mixture in the presence of a re-oxidizing agent, such as trimethylamine N-oxide, at room temperature for one hour to three days. Note that the reaction time varies depending upon the reaction conditions.

By the esterification of Step H, a sulfoquinovosyldiacylglycerol derivative having desired higher fatty acids each bonded, through an ester-bond, to its glycerol moiety can be obtained.

This reaction can be carried out by adding a higher fatty acid corresponding to a final product to a solution of the compound 8 dissolved in a suitable organic solvent, such as dichloromethane, and then reacting the resultant mixture, if necessary, in the presence of a suitable catalyst, such as ethyldimethylaminopropylcarbodiimide (EDCI)-DMAP.

In the reaction of Step H, as the fatty acid to be added, use may be made of a higher fatty acid whose acyl group is that represented by R₁₀₁ of Formula (1), i.e., a straight-chain or branched-chain, saturated or unsaturated higher fatty acid.

In the reaction of Step H, the compound 9 is obtained in the form of a mixture of a diacylester and a monoacylester. The diacylester herein is represented by Formula (1) of the present invention where each of R₁₀₁ and R₁₀₂ is an acyl residue of the higher fatty acid added. The monoacylester herein has the acyl residue of the higher fatty acid added, as the R₁₀₁ only. Two or more higher fatty acids may be added, if desired, in the reaction of Step H. In this case, the resultant mixture contains diacylesters represented by Formula (1) where R₁₀₁ and R₁₀₂ are the same or different acyl residues, and monoesters having different acyl residues as R₁₀₁.

If necessary, the mixture of the monoesters and diesters can be isolated from each other by, for example, chromatography, and subjected to the next reaction Step I. Furthermore, production of the monoester is suppressed as much as possible by setting the addition amount of the fatty acid to 2-3 times larger than that of the compound 8, in terms of mole, thereby the diester can be preferentially obtained.

In Step I, the conversion into a sulfonate salt can be carried out by adding an oxidizing agent, for example, OXONE (2KHSO₅, KHSO₄ and K₂SO₄) into a solution of the compound 9 dissolved in an organic solvent, which is buffered with acetic acid and potassium acetate, and then allowing the resultant mixture to react at room temperature for a half day to two days. Note that the reaction time varies depending upon the reaction conditions.

The deprotection of the protecting groups bonded to carbons at the C2 to C4 carbons in Step J can be carried out by a method suitable for a protecting group to be used and an acyl residue of the bonded higher fatty acid. For example, when the protecting group is a benzyl group and each of R₁₀₁ and R₁₀₂ is an acyl residue of a saturated higher fatty acid, the deprotection can be conducted by reacting a solution of a compound 10 dissolved in an organic solvent, such as ethanol, in the presence of a catalyst, such as palladium-activated carbon, under a hydrogen gas atmosphere at room temperature. Furthermore, when at least one of the acyl residues of the higher fatty acids represented by R₁₀₁ and R₁₀₂ is an acyl residue of an unsaturated higher fatty acid, a deprotection method suitable for a protecting group used and capable of retaining the double bond of the unsaturated fatty acid may be employed. For example, when the protecting group is a silyl group, the deprotection can be conducted by use of an acid catalyst (e.g., trifluoroacetic acid).

Note that glucose of a starting material usually takes α- and β-anomer configurations in a solution. Therefore, the product in each step results in a mixture of α- and β-anomers. The mixture may be separated into α- and β-anomers by chromatography.

Prior to the step B, if a step of reacting the compound 2 with benzaldehyde is performed to form benzylidene, it is possible to selectively crystallize the α-anomer and thereby separate it. Furthermore, if halogen such as bromin is bonded to the C1 carbon before the 2-propenylation of Step A, the propenyl group can be introduced into a β-configuration in a later reaction. In this way, a β-anomers can be selectively synthesized.

The immunosuppressive agent of the present invention contains at least one compound selected from sulfoquinovosyldiacylglycerol derivatives represented by Formula (1) of the present invention and pharmaceutically acceptable salts thereof, as an active ingredient. Examples of the pharmaceutically acceptable salts employed in the immunosuppressive agent of the present invention include, but not limited to, a salt of a monovalent cation such as a sodium or potassium ion. Hereinafter, the compounds of the group consisting of sulfoquinovosyldiacylglycerol derivatives and pharmaceutically acceptable salts thereof are sometimes referred to as "immunosuppressive substance of the present invention".

Examples of the sulfoquinovosylacylglycerol derivative contained in the immunosuppressive substance of the present invention as an active ingredient include an isomer in which quinovose is bonded to glycerol with an α- or β-configuration, and an isomer having an asymmetric carbon at the C2 carbon of the glycerol moiety. The immunosuppressive substance of the present invention may include these isomers alone or in combination of two or more types of isomers as long as they adversely affect the activity of the immunosuppressive substance.

The immunosuppressive substance of the present invention can be orally or parenterally administered. Immunosuppressive substance of the present invention can be combined with, for example, a pharmaceutically acceptable excipient or diluent depending on an administration route thereby to form a medicinal formulation.

The forms of the agent suitable for oral administration include, solid-, semi-solid, liquid- and gas-states. Specific examples include, but not limited to, tablet, capsule, powder, granule, solution, suspension, syrup and elixir agents.

In order to formulate the immunosuppressive substance of the present invention into tablets, capsules, powders, granules, solutions or suspensions, the substance is mixed with a binder, a disintegrating agent and/or a lubricant, and, if necessary, the resultant is mixed with a diluent, a buffer, a wetting agent, a preservative and/or a flavor, by a known method. Examples of the binder include crystalline cellulose, cellulose derivatives, cornstarch and gelatin. Examples of the disintegrating agent include cornstarch, potato starch and sodium carboxymethylcellulose. Examples of the lubricant include talc and magnesium stearate. Furthermore, additives such as lactose and mannitol may also be used as long as they are used conventionally.

Moreover, the immunosuppressive substance of the present invention may be administered in the form of aerosol or inhalant, which is prepared by charging the active substance of liquid- or fine powder-form, together with a gaseous or liquid spraying agent, and, if necessary, a known auxiliary agent such as a wetting agent, into a non-pressurized container such as an aerosol container or a nebulizer. As the spraying agent, a pressurized gas, for example, dichlorofluoromethane, propane or nitrogen may be used.

For parenteral administration, the immunosuppressive substance of the present invention can be administered by injection, percutaneously, rectally or intraocularly.

For the administration by injection, the immunosuppressive substance of the present invention can be injected, for example, hypodermically, intracutaneously, intravenously or intramuscularly. An injection preparation may be formulated by dissolving, suspending or emulsifying the immunosuppressive substance of the present invention into an aqueous or non-aqueous solvent such as a vegetable oil, a synthetic glyceride with a fatty acid, an ester of a higher fatty acid or propylene glycol by a known method. If desired, a conventional additive such as a solubilizing agent, an osmoregulating agent, an emulsifier, a stabilizer or a preservative, may be added to the preparation.

For formulating the immunosuppressive substance of the present invention into solutions, suspensions, syrups or elixirs, a pharmaceutically acceptable solvent such as sterilized water for injection or a normalized physiological saline solution may be used.

For the percutaneous administration, the immunosuppressive substance of the present invention may be administered in the form of ointment, emulsifications, pastae, plasters, liniments, lotions, suspensions in accordance with the state of skin to be treated.

The ointments can be formulated by a known method by kneading the immunosuppressive substance of the present invention with a hydrophobic base, such as Vaseline or paraffin, or a hydrophilic bas, such as hydrophilic Vaseline or macrogol. The emulsifying agents and other percutaneous agents may be formulated by a method conventionally used.

For the rectal administration, a suppository can be used. The suppository may be prepared by mixing the immunosuppressive substance of the present invention with an excipient that can be melted at body temperature but is solid at room temperature, such as cacao butter, carbon wax or polyethylene glycol, and molding the resultant material, by a known method.

For the intraocular administration, ophthalmic formulations such as eye drops and eye ointments may be administered. The eye drops are formulated by dissolving or suspending the immunosuppressive substance of the present invention in an aqueous solvent, such as sterilized water, and, if necessary, adding a preservative, buffer, and surfactant.

The immunosuppressive substance of the present invention may be used together with a pharmaceutically acceptable compound having another activity, to prepare a pharmaceutical preparation.

The dose of the immunosuppressive substance of the present invention may be appropriately set or adjusted in accordance with an administration form, an administration route, a degree or stage of a target disease, and the like. For example, in the case of oral administration, a dose of the immunosuppressive substance may be set at 1 - 100 mg/kg body weight/day, preferably 1 - 10 mg/kg body weight/day. In the case of administration by injection, a dose of the immunosuppressive substance may be set at 1 - 50 mg/kg body weight/day, more preferably, 1 - 5 mg/kg body weight/day. In the case of percutaneous administration, a dose of the immunosuppressive substance may be set at 1 - 100 mg/kg body weight/day, more preferably, 1 - 10 mg/kg body weight/day. In the case of rectal administration, a dose of the immunosuppressive substance may be set at 1 - 50 mg/kg body weight/day, more preferably 1 - 5 mg/kg body weight/day. In the case of intraocular administration, about a 0.01 - 3% solution of the immunosuppressive substance may be applied dropwise to an eye several times per day. However, the doses are not limited to these.

### Examples

The present invention will now be described by way of its Examples.

Synthesis example of a sulfoquinovosyldiacylglycerol β derivative is set forth below, as an example of the active ingredients used in the immunosuppressive substance of the present invention.

### <Example 1>

### Route a: 2,3,4,6-tetra-O-acetyl-D-glucopyranosyl bromide (II)

To 400 mL of acetic anhydride, 2.4 mL of 60% perchloric acid was added dropwise at 0°C. After the solution was returned to room temperature, 100g (0.56 mol) of D-glucose was added to the solution with stirring for about 30 minutes while keeping the solution temperature at 30-40°C. After the reaction solution was cooled to 20°C, 30g (1.0 mol) of red phosphorus was added. While the solution temperature was maintained at 20°C or less, 180g (2.3 mol) of bromine, and then 36 mL of water were added dropwise. After the resultant mixture was allowed to stand at room temperature for 2 hours, 300 mL of cold chloroform was added to the mixture. The reaction solution was filtrated by a funnel having a glass wool placed at the bottom. The filtrate was poured into cold water (800 mL) and a chloroform layer was separated by a separatory funnel. The water layer was extracted with 50 mL of chloroform. The chloroform layers were combined and washed with cold water (300 mL). The resultant chloroform layer was poured into 500 mL of saturated sodium hydrogencarbonate solution, sufficiently shaken in a separatory funnel. The resultant chloroform layer was recovered, dried over anhydrous sodium sulfate, filtrated, and concentrated in vacuo to give a crystalline substance. The obtained crystalline substance was pulverized in a mortar together with a solution of petroleum ether : ether (2:1) and filtered off. The crude crystalline substance was dried in vacuo, recrystallized with cold diisopropylether. As a result, a pure crystal was obtained (yield: 152.6g, 0.37 mol, recovery: 66.7%).

Melting point: 88-90°C. Rf value: 0.338 (Hexan: ethyl acetate=4:1)

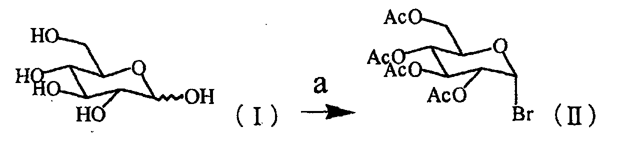

### Route b: 2,3,4,6-tetra-O-acetyl-1-0-(2-propenyl)-β-D-glucose (III)

In 100 mL of allyl alcohol, 16.7g (40.6 mmol) of the compound (II) was dissolved, and 10.0g (39.6 mmol) of mercury cyanide was added thereto. The resultant mixture was stirred at room temperature overnight. The reaction solution was concentrated in vacuo, shaken together with 100 mL of chloroform and cold water in a separatory funnel to allow the chloroform layer to separate. After the chloroform layer was dried over anhydrous sodium sulfate, filtrated, and concentrated in vacuo, the resultant syrup was dissolved in cold diisopropylether. To the resultant solution, a small amount of crystal seed was added and stood in an ice-cooled condition. As a result, a crystal was obtained (yield: 12.6g, 32.5 mmol, recovery: 80%)

Melting point: 77-81°C, Rf value: 0.282 (benzene: ethyl acetate = 4:1)

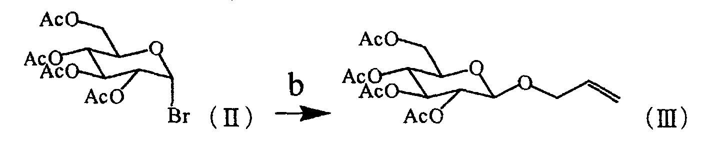

### Route c: 1-0-(2-propenyl)-β-D-glucose (IV)

The compound (III)(30.3g, 78.1 mmol) was dissolved in 120 mL of methanol. To the solution mixture, a small amount of 28% sodium methoxide in methanol was added dropwise while stirring. The reaction mixture was reacted at room temperature for 4 hours, neutralized with 0.1N hydrochloric acid, dried over anhydrous sodium sulfate, filtrated, concentrated in vacuo, and purified by silica gel flash chromatography (chloroform : methanol = 4 : 1). As a result, a colorless and transparent oily substance was obtained (yield: 15.8g, 71.8 mmol, recovery: 91.9%).
Rf value: 0.407 (Chloroform : methanol = 4 : 1)

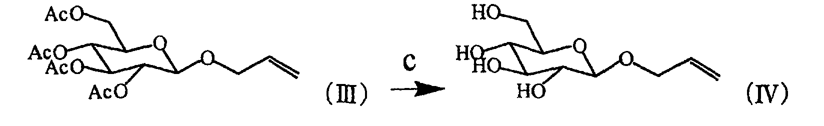

### Route d: 1-0-(2-propenyl)-6-0-triphenylmethyl-β-D-glucose (V)

The compound (IV)(15.8g, 71.8 mmol) was dissolved in 120 mL of anhydrous pyridine. To the solution, 23.4g (83.9 mmol) of trityl chloride and 0.1g (0.82 mmol) of p-dimethylaminopyridine (DMAP) were added. The reaction mixture was reacted for 36 hours at room temperature while stirring. Then, the reaction was quenched by adding 300 mL of cold water and the extracted with ethyl acetate (3×300 mL). The resultant organic layers were combined, neutralized with 1.0N hydrochloric acid to pH 4, washed with brine (2×300 mL), dried over anhydrous sodium sulfate, filtered, concentrated in vacuo, and purified by silica gel flash chromatography (dichloromethane: methanol = 20 : 1)). As a result, a pale yellowish oily substance was obtained (yield: 28.7g, 62.1 mmol, recovery: 86.5%). Rf value: 0.306 (chloroform : methanol= 19 : 1).

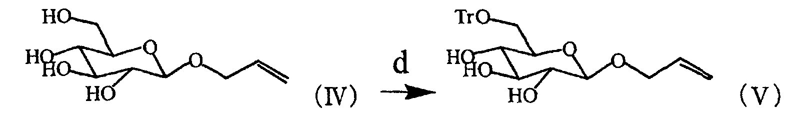

### Route e: 2,3,4-tri-O-benzyl-1-0-(2-propenyl)-6-0-triphenylmethyl-β-D-glucose (VI)

80% sodium hydride (3.2g, 133 mmol) dispersed in a mineral oil was put into a reaction vessel, and sufficiently washed with anhydrous hexane (50 mL). After the hexane was removed, 14.2g (30.7 mmol) of the compound (V) dissolved in dry N,N-dimethylformamide was gradually added to the resultant suspension in an ice-cooled condition. After 15 minutes, the reaction mixture was returned to room temperature and the reaction was performed for one hour while stirring.

Next, 21.6g (126 mmol) of benzylbromide were gradually added to the reaction mixture under an ice-cooled condition again. After 15 minutes, the reaction mixture was returned to room temperature, and reacted for 3 hours while stirring. Then, 20 mL of methanol and 30 mL of cold water were added to the reaction mixture to quench the reaction. The reaction mixture was extracted with ethyl acetate (3×50 mL). The organic layers were combined, washed with brine (2×100 mL), dried over anhydrous sodium sulfate, filtered, concentrated in vacuo, and purified by silica gel flash chromatography (hexane : ethyl acetate = 10 : 1). As a result, a pale yellowish oily substance was obtained (yield: 21.6g, 29.5 mmol, recovery: 96.1%). The Rf value: 0.410 (hexane : ethyl acetate = 4 : 1)

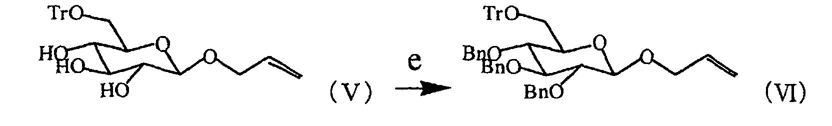

### Route f: 2,3,4-tri-O-benzyl-1-0-(2-propenyl)-β-D-glucose (VII)

In 150 mL of methanol, the compound (VI) (21.6g, 29.5 mmol) was dissolved and 2.80g (14.7 mmol) of p-toluenesulfonic acid monohydrate was added. The solution was reacted overnight while stirring. Thereafter, the reaction was quenched by adding 100 mL of cold water and extracted with ethyl acetate (3×200 mL). The organic layers were combined, washed with brine (2×300 mL), dried over anhydrous sodium sulfate, filtered, concentrated in vacuo, and purified to silica gel flash chromatography (hexane : ethyl acetate = 4 : 1). As a result, a white crystalline substrate was obtained (yield 9.0g, 18.4 mmol, recovery 62.4%).

Melting point: 80-82°C,

Rf value: 0.338 (hexane : ethyl acetate = 4:1) [α]_{D} = +0.4° (c 5.50 CHCl₃)

¹H NMR (300MHz, CDCl₃+TMS, δ) ; 7.36-7.23 (15H, m, Ar), 6.02-5.89 (1H, m, -CH=CH₂), 5.34 (1H, dd, J=1.5 & 17.2, -CH=CH₂), 5.22 (1H, dd, J=1.4 & 10.4, -CH=CH₂), 4.95 (1H, d, J=10.9, Ar-CH₂), 4.94 (1H, d, J=10.9, Ar-CH₂), 4.86 (1H, d, J=10.9, Ar-CH₂), 4.81 (1H, d, J=10.9, Ar-CH₂), 4.73 (1H, d, J=10.9, Ar-CH₂), 4.64 (1H, d, J=10.9, Ar-CH₂), 4.50 (1H, d, J=7.8, H-1), 4.43-4.13 (2H, m, -O-CH₂-CH=CH₂), 3.88-3.34 (6H, m, H-2 & H-3 & H-4 & H-5 & H-6a,b)

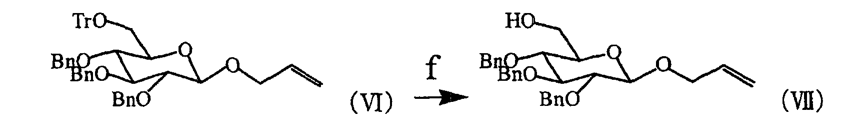

### Route g: 2,3,4-tri-O-benzyl-1-0-(2-propenyl)-6-0-(4-tolylsulfonyl)-β-D-glucose (VIII)

In 200 mL of anhydrous pyridine, 9.80g (20.0 mmol) of the compound (VII) was dissolved, and then 24.4 mg (0.2 mmol) of DMAP and 11.4g (60.0 mmol) of p-toluenesulfonyl chloride were added. The solution was reacted at room temperature overnight while stirring. Thereafter, the reaction was quenched by adding 300 mL of cold water and extracted with ethyl acetate (3×200 mL). The resultant organic layers were combined, neutralized to pH 4 with 1.0N and 0.1N hydrochloric acid, washed with brine (2×300 mL), dried over anhydrous sodium sulfate, filtered, concentrated in vacuo, and purified by silica gel flash chromatography (hexane : ethyl acetate = 4 : 1). As a result, a white crystalline substance was obtained (yield: 9.00g, 14.0 mmol, recovery 70.0%).

Melting point: 111-112°C, Rf value: 0.295 (hexane: ethyl acetate = 4 : 1)

¹H NMR (300MHz, CDCl₃+TMS, δ) ;7.77 (2H, d, J=8.2, H at Ts Me), 7.31-7.25 (15H, m, Ar), 7.19-7.16 (2H, m, H at Ts SO₂), 5.98-5.85 (1H, m, -CH=CH₂), 5.35-5.19 (2H, m, -CH=CH₂), 4.92 (2H, d, J=10.9, Ar-CH₂), 4.81 (1H, d, J=10.8, Ar-CH₂), 4.75 (1H, d, J=10.9, Ar-CH₂), 4.68 (2H, d, J=10.9, Ar-CH₂), 4.48 (1H, d, J=10.8, Ar-CH₂), 4.39 (1H, d, J=7.8, H-1), 4.34-3.38 (8H, m, H-2 & H-3 & H-4 & H-5 & H-6a,b & -O-CH₂-CH=CH₂), 2.42 (3H, s, Ts CH₃)

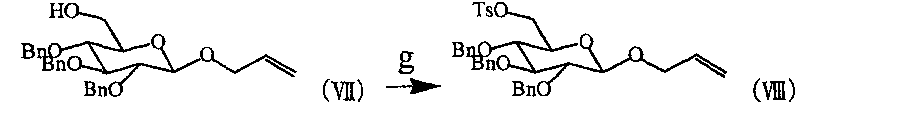

### Route h: 2,3,4-tri-O-benzyl-1-O-(2-propenyl)-6-deoxy-6-acetylthio-β-D-glucose (IX)

In 250 mL of anhydrous ethanol, 9.00g (14.0 mmol) of the compound (VIII) was dissolved and then 4.80g (42.0 mmol) of potassium thioacetate was added. The solution was reacted under reflux for 3 hours while stirring. Thereafter, the reaction was quenched by adding 300 mL of cold water, and extracted with ethyl acetate (3×200 mL). The organic layers were combined, washed with brine (2×300 mL) , dried over anhydrous sodium sulfate, filtered, concentrated in vacuo, purified by silica gel flash chromatography (hexane : ethyl acetate = 10 : 1). As a result, a white crystalline substance was obtained (yield: 6.60g, 12.0 mmol, recovery: 85.7%). Melting point: 70-73°C, Rf value: 0.295 (hexane : ethyl acetate= 4:1)
[α]_{D} = +26.7(c 0.75, CHCl₃)

¹H NMR (300MHz, CDCl₃+TMS, δ) ; 7.35-7.25 (15H, m, Ar), 6.00-5.89 (1H, m, -CH=CH₂), 5.35 (1H, dd, J=1.5 & 17.2, -CH=CH₂), 5.22 (1H, dd, J=1.3 & 10.4, -CH=CH₂), 4.95 (1H, d, J=10.9, Ar-CH₂), 4.93 (1H, d, J=10.8, Ar-CH₂), 4.87 (1H, d, J=10.8, Ar-CH₂), 4.78 (1H, d, J=10.9, Ar-CH₂), 4.71 (1H, d, J=10.9, Ar-CH₂), 4.63 (1H, d, J=10.7, Ar-CH₂), 4.42 (1H, d, J=7.9, H-1), 4.37-3.33 (7H, m, H-2 & H-3 & H-4 & H-5 & H-6a & -O-CH₂-CH=CH₂), 2.96 (1H, dd, J=6.9 & 13.6, H-6b) , 2.34 (3H, s, SCOCH₃)

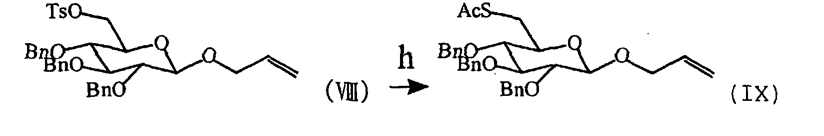

### Route i: 3-0-(2,3,4-tri-O-benzyl-6-deoxy-6-acetylthio-β-D-glucopyranosyl)-glycerol (X)

In a mixture of t-butanol : H₂O (4 : 1), 3.30g (6.02 mmol) of the compound (IX) was dissolved and then 1.34g (12.1 mmol) of trimethylamine N-oxide dihydrate and 1.7 mL of 0.04 M solution of osmium tetraoxide in t-butanol were added. The solution was reacted at room temperature for 3 days while stirring. Thereafter, 5.8g of activated charcoal was added, and then the reaction mixture was allowed to stand while stirring for 1.5 hours. After filtration with suction, the reaction was quenched by adding 250 mL of cold water and extracted with ethyl acetate (3×200 mL). The organic layers were combined, washed with brine (2×300 mL), dried over anhydrous sodium sulfate, filtered, concentrated in vacuo, and purified by silica gel flash chromatography (hexane : ethyl acetate = 1 : 1). As a result, a white crystalline substance was obtained (yield: 1.79g, 3.08 mmol, recovery 51.2%). Melting point: 91-93°C, Rf value: 0.112 (hexane : ethyl acetate = 1 : 1) , [α]_{D} = +18.0 (c 0.75, CHCl₃)

¹H NMR (300MHz, CDCl₃+TMS, δ) ;7.35-7.25 (15H, m, Ar), 4.94-4.61 (6H, m, Ar-CH₂), 4.38 (0.5H, d, J=7.8, H-1(R or S)), 4.37 (0.5H, d, J=7.8, H-1(R or S)), 3.83-3.37 (7H, m, H-2 & H-3 & H-4 & H-5 & H-6a & -O-CH₂-CH=CH₂), 2.96 (1H, dd, J=6.9 & 13.6, H-6b), 2.34 (3H, s, SCOCH₃)

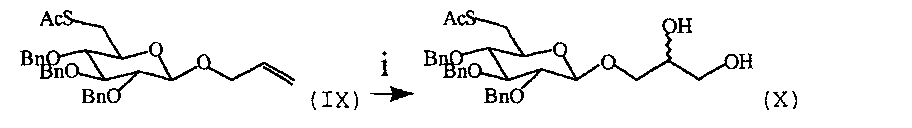

### Route j: 3-O-(2,3,4-tri-O-benzyl-6-deoxy-6-acetylthio-β-D-glucopyranosyl)-1,2-di-O-stearoyl-glycerol (XI)

Into 100 mL of dichloromethane, 1.23g (2.12 mmol) of the compound (X) was dissolved and then 1.30g (6.79 mmol) of 1-ethyl-3-(3-dimethylaminopropyl)-carbodiimide hydrochloride (EDCI), 260 mg (2.13 mmol) of DMAP, and 1.75g (6.15 mmol) of stearic acid, were added. The solution was reacted at room temperature for a day while stirring. Thereafter, the reaction was quenched by adding 100 mL of dichloromethane, washed with brine (2×50 mL), dried over anhydrous sodium sulfate, filtered, concentrated in vacuo, and purified by silica gel flash chromatography (hexane : ethyl acetate = 10 : 1 → 8 : 1). As a result, a white non-crystalline solid substance was obtained (yield: 2.10g, 1.88 mmol, recovery: 88.7%). Rf value: 0.487 (hexane; ethyl acetate = 4 : 1)
[α]_{D} = -21.3 (c 0.15, CHCl₃)

¹H NMR (300MHz, CDCl₃+TMS, δ) ;7.34-7.26 (15H, m, Ar), 5.28(1H, m, Gly-H-2), 4.92-4.60 (6H, m, Ar-CH₂), 4.36 (0.5H, d, J=7.7, H-1(R or S)), 4.35 (0.5H, d, J=7.8, H-1(R or S)), 4.25-2.96 (10H, m, H-2 & H-3 & H-4 & H-5 & H-6a, b & Gly-H-1a, b & Gly-H-3a, b), 2.37 (1.5H, s, SCOCH₃(R or S)), 2.35 (1.5H, s, SCOCH₃(R or S)), 2.32-2.24(4H, m, OCOCH₂), 1.65-1.58 (4H, m, OCOCH₂CH₂), 1.25 (56H, br, -CH₂-), 0.88 (6H, t, J=6.3, CH₃) 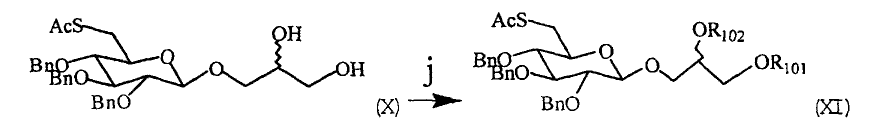 R₁₀₁=R₁₀₂=stealoyl

### Route k: 3-O-(2,3,4-tri-O-benzyl-6-deoxy-6-sulfo-β-D-glucopyranosyl)-1,2-di-O-stealoyl-glycerol sodium salt (XII)

In 50 mL of acetic acid, 1.24g
(1.11 mmol) of the compound (XI) was dissolved and then 1.5g of potassium acetate and 1.55g of OXONE (2KHSO₅, KHSO₄, K₂SO₄) were added. The solution was reacted overnight at room temperature while stirring. Thereafter, the reaction was quenched by adding 100 mL of cold water, and extracted with ethyl acetate (5×50 mL). The organic layers were combined, neutralized with saturated sodium hydrogencarbonate (2×100 mL), washed with brine (2×100 mL), dried over anhydrous sodium sulfate, filtered, concentrated in vacuo, and purified by silica gel flash chromatography (chloroform : methanol = 20 : 1 → 15 : 1). As a result, a white non-crystalline solid substance was obtained (yield: 773 mg, 0.677 mmol, recovery 61.0%). Rf value: 0.288 (dichlormethane: methanol = 10 : 1), [α]_{D} = +4.8° (c 0.17, CHCl₃)

¹H NMR (300MHz, CDCl₃+TMS, δ) ; 7.29-7.16 (15H, m, Ar), 5.31(1H, m, Gly-H-2), 4.91-4.56 (6H, m, Ar-CH₂), 4.50 (1H, d, J=7.6, H-1), 4.44-3.03 (10H, m, H-2 & H-3 & H-4 & H-5 & H-6a, b & Gly-H-1a, b & Gly-H-3a, b), 2.62(4H, br, OCOCH₂), 2.19-2.17 (4H, br, OCOCH₂CH₂), 1.49 (4H, br, OCOCH₂CH₂CH₂), 1.25 (52H, br, -CH₂-), 0.88 (6H, t, J=6.3, CH₃) 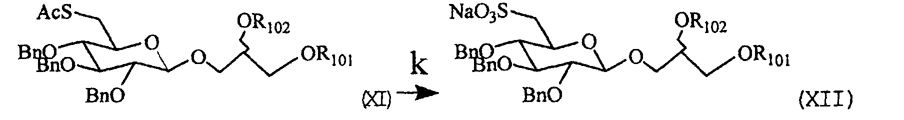 R₁₀₁=R₁₀₂=stearoyl

### Route l: 3-O-(6-deoxy-6-sulfo-β-D-glucopyranosyl)-1,2-di-O-stearoyl-glycerol sodium salt (XIII)

Into 50 mL of ethanol, 773 mg (0.677 mmol) of the compound (XII) was dissolved and then 2.00g of 10% palladium-activated carbon (Pd-C) was added. After the atmosphere of the flask was substituted with hydrogen, the solution mixture was reacted at room temperature overnight while stirring. Then, the reaction mixture was filtered with suction using celite, concentrated in vacuo, and purified by silica gel flash chromatography (chloroform: methanol = 10 : 1 → chloroform: methanol: water = 70 : 30 : 4). As a result, a white non-crystalline solid substance was obtained (yield: 311 mg, 0.356 mmol, recovery 52.6%).

Rf value: 0.402 (chloroform : methanol : water = 65 : 25 : 4), [α]_{D} = -3.6° (c 0.59, CHCl₃: CH₃OH:H₂O = 70:30:4).

¹H NMR (300MHz, CDCl₃+TMS, δ) ; 5.28(1H, m, Gly-H-2), 4.32 (1H, d, J=7.7, H-1), 4.27-3.11 (10H, m, H-2 & H-3 & H-4 & H-5 & H-6a, b & Gly-H-1a, b & Gly-H-3a, b), 2.36-2.30(4H, m, OCOCH₂), 1.60 (4H, m, OCOCH₂CH₂), 1.27 (56H, br, -CH₂-), 0.89 (6H, t, J=6.4, CH₃) 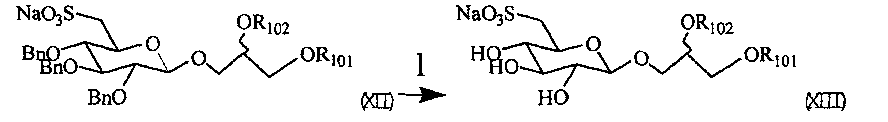 R₁₀₁=R₁₀₂=stearoyl

### <Assay 1>

### Mixed lymphocytes reaction

Lymphocytes serving as stimulator cells and responder cells were prepared from blood taken from individual healthy persons.

The responder cells were further separated from the lymphocyte cells to give T lymphocytes alone.

No treatment was applied to the responder cells. 10⁶/mL of the stimulator cells were treated with 10 µg/mL of mitomycin C to stop the cell growth. Thereafter, the stimulator cells were washed with PBS (phosphate buffer saline) 4 times.

Subsequently, the responder cells were inoculated at a rate of 10⁵ cells per well and then test substances (compounds SQAG 3, SQAG 5, SQAG 7, and SQAG 9 listed in Table 1 below) were added to a predetermined concentration. The reaction mixture was cultured at 37°C for one hour. Thereafter, the stimulator cells were added at a rate of 10⁵ cells per well. The mixture was cultured in a CO₂ incubator at 37°C for 4 days. After the incubation, the proliferation ability of the responder cells was quantified as follows. First, [³H]-thymidine was added to the responder cells and incorporated into the nucleus of the cells by culturing the cells for 12 hours. Then, the amount of [³H]-thymidine uptake into the cells was determined by a scintillation counter.

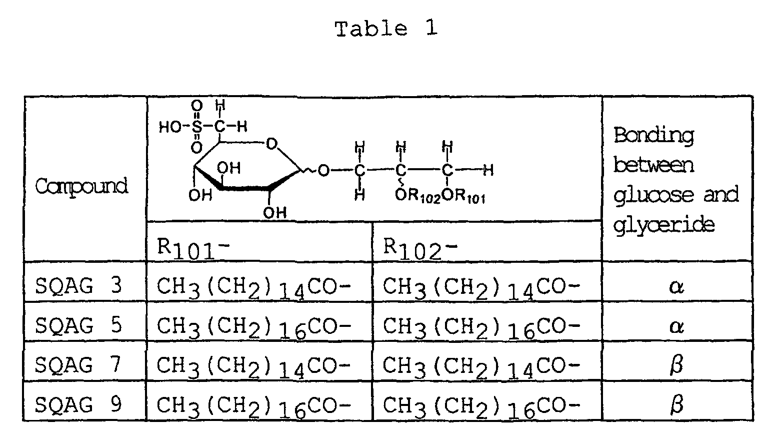

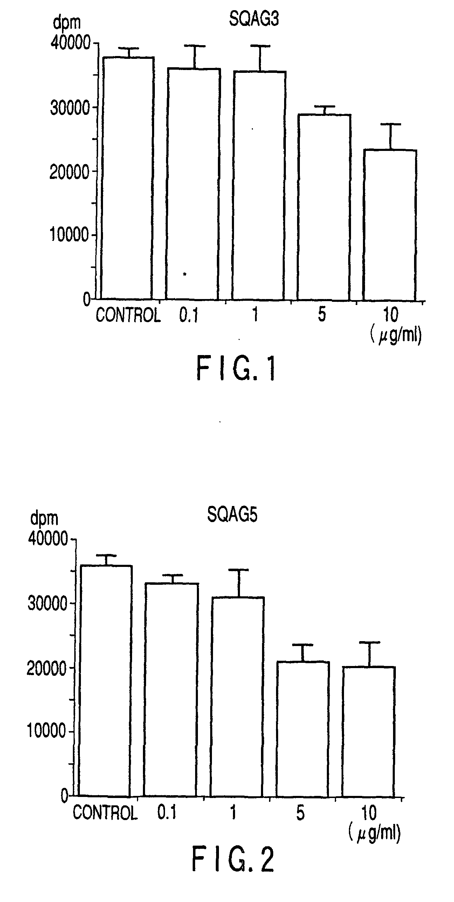

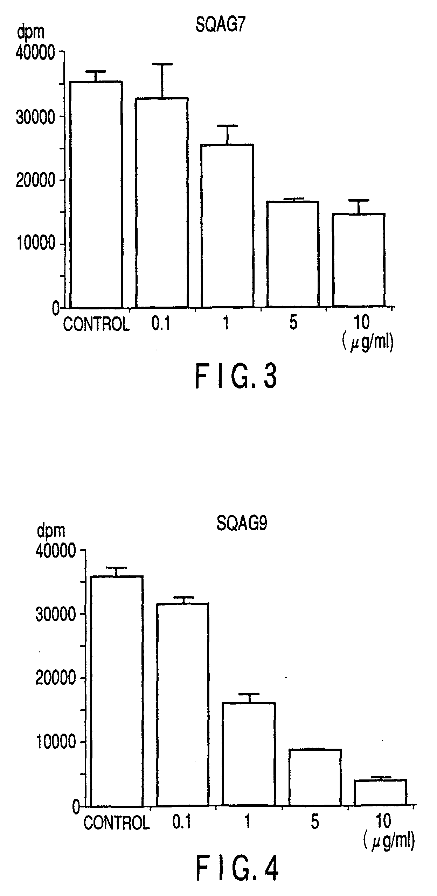

The results are shown in FIGS. 1-4.

FIGS. 1-4 show the amounts of [³H]-thymidine uptake when SQAG 3, SQAG 5, SQAG 7, and SQAG 9 are added. The lower the amount of [³H]-thymidine uptake, the higher the immunosuppressive activity. In each of the FIGS. 1-4, the vertical axis indicates the intensity of radioactivity and the horizontal axis indicates the concentration of the test substance.

As is apparent from FIGS. 1-4, all test substances have significant immunosuppressive activities. In particular, SQAG 9 has a considerably higher immunosuppressive activity than other compounds.

### <Assay 2>

### Rejection test in skin graft

ACI rats and LEW rats were prepared and subjected to inhalation anesthesia of ether. After shaving the dorsum of each ACI rat, posterior lateral skin (1×1 cm) was removed to form a skin-flayed wound, and then a hemostatic treatment was performed. On the other hand, a piece of skin (1×1 cm) was obtained from the tail of each LEW rat. The donor skin was applied to the skin-flayed wound of each ACI rat and sutured with 5-0 nylon sutures. Then, gauze was applied to the surgically operated portion and a corset with a rubber bandage was further attached thereon to protect it. Such a skin transplantation operation was applied to all ten ACI rats in the same manner. Of the ten ACT rats, five rats were selected at random and classified as a control group, and the remaining five rats were classified as a test group to which test substances are to be administered. To the control group, 10 mL of physiological saline was intraperitoneally injected daily for five days after the surgery. To the test group, 10 mL solution of SQAG 9 dissolved in physiological saline at a concentration of 1 mg/mL was intraperitoneally injected daily for five days after the surgery in the same manner as in the control group.

On the seventh day after the operation, a graft was taken to prepare a tissue specimen. Subsequently, H-E staining was performed in accordance with the method of Fujita et al. (T. Fujita, S. Takahashi, A. Yagihashi, K. Jimbow, N. Sato, Transplantation, vol. 64, 922-925, 1997). The results were histologically examined.

As a result of histological observation, it was confirmed that the epidermal layer was removed from the dermis in all five rats of the control group, and that necrosis of the epidermis had occurred. In contrast, necrosis of the epidermal layer was observed in three out of five rats in the SQAG 9 administered group. However, in the remaining two rats, it was observed that some parts of the epidermis successfully adhered to the derims even though other parts of the epidermal layer were partly removed from the dermis.

The present inventors consider that the skin graft rejection test is the most stringent method of evaluation for an immunosuppressive activity. It is considered that rejection occurred most severely when transplantation was made between the ACI series rats and the LEW series rats, as used in this assay. However, under such stringent conditions, the activity for suppressing the rejection was observed in two out of five rats in this assay. This fact demonstrates that SQAGs of the present invention are highly effective as an immumosuppressive agent.

In addition, none of the rats administered with SQAGs died from acute toxicity during the test period. Neither weight reduction nor malphysical conditions were visually observed. No abnormality was observed with respect to major organs in general pathological tests. It is therefore demonstrated that the immunosuppressive agents of the present invention are extremely low in toxicity.

Of the commercially available immunosuppressive agents, a few (e.g., FK506) are known as effective in suppressing rejection in skin graft tests. However, highly effective immunosuppressive agents low in toxicity have not yet been reported.

## Claims

1. The use of a compound selected from compounds represented by Formula (1): 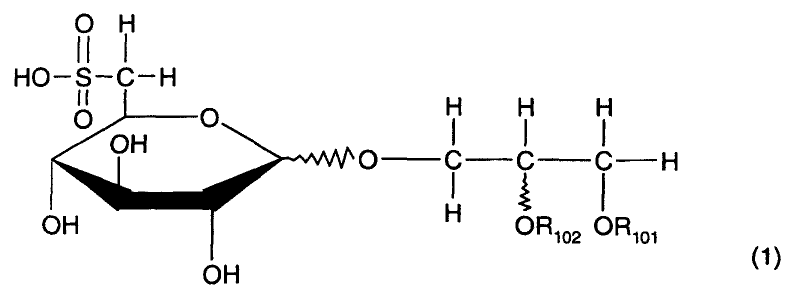 where R₁₀₁ and R₁₀₂ independently represent an acyl residue of a higher fatty acid represented by R-C(=O), wherein R represents an alkyl or alkenyl group having 13 or more carbon atoms, and
pharmaceutically acceptable salts thereof in the manufacture of a medicament for the suppression of an immune response.

2. The use according to claim 1 , wherein the bonding between the sulfoquinovosyl moiety and the glyceryl moiety in the formula (1) is in the α-anomeric configuration.

3. The use according to claim 1, wherein the bonding between the sulfoquinovosyl moiety and the glyceryl moiety in the formula (1) is in the β-anomeric configuration.

4. The use according to any one of claims 1 to 3, wherein R represents an alkyl group.

5. The use according to claim 4, wherein R has 13 to 25 carbon atoms.

6. The use according to claim 5, wherein both R₁₀₁ and R₁₀₂ are CH₃(CH₂)₁₄CO-.

7. The use according to claim 5, wherein both R₁₀₁ and R₁₀₂ are CH₃(CH₂)₁₆CO-.

8. The use according to any one of claims 1 to 3, wherein R represents an alkenyl group.

9. The use according to claim 8, wherein R has 13 to 25 carbon atoms.

## Patentansprüche

1. Verwendung einer Verbindung, die aus Verbindungen ausgewählt ist, welche durch die Formel (1): 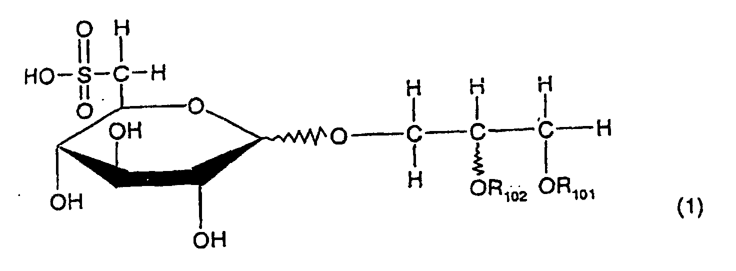 repräsentiert werden, wobei R₁₀₁ und R₁₀₂ unabhängig einen Acylrest einer höheren Fettsäure, repräsentiert durch R-C(=O), wobei R eine Alkyl- oder Alkenylgruppe mit 13 oder mehr Kohlenstoffatomen repräsentiert, repräsentieren, und pharmazeutisch annehmbarer Salze davon zur Herstellung eines Medikaments zur Unterdrückung einer Immunreaktion.

2. Verwendung nach Anspruch 1, wobei die Bindung zwischen der Sulfochinovosyl-Gruppierung und der Glyceryl-Gruppierung in der Formel (1) in der α-anomeren Konfiguration vorliegt.

3. Verwendung nach Anspruch 1, wobei die Bindung zwischen der Sulfochinovosyl-Gruppierung und der Glyceryl-Gruppierung in der Formel (1) in der β-anomeren Konfiguration vorliegt.

4. Verwendung nach irgendeinem der Ansprüche 1 bis 3, wobei R eine Alkylgruppe repräsentiert.

5. Verwendung nach Anspruch 4, wobei R 13 bis 25 Kohlenstoffatome aufweist.

6. Verwendung nach Anspruch 5, wobei R₁₀₁ und R₁₀₂ beide CH₃(CH₂)₁₄CO- sind.

7. Verwendung nach Anspruch 5, wobei R₁₀₁ und R₁₀₂ beide CH₃(CH₂)₁₆CO- sind.

8. Verwendung nach irgendeinem der Ansprüche 1 bis 3, wobei R eine Alkenylgruppe repräsentiert.

9. Verwendung nach Anspruch 8, wobei R 13 bis 25 Kohlenstoffatome aufweist.

## Revendications

1. Utilisation d'un composé choisi parmi les composés représentés par la formule (1) : 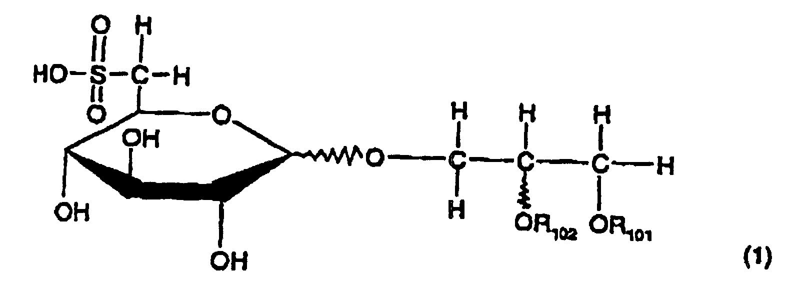 dans laquelle R₁₀₁ et R₁₀₂ représentent indépendamment un résidu acyle d'un acide gras supérieur représenté par R-C(=O), dans lequel R représente un groupe alkyle ou alcényle ayant 13 ou plus atomes de carbone, et
leurs sels pharmaceutiquement acceptables dans la fabrication d'un médicament pour la suppression d'une réponse immune.

2. Utilisation selon la revendication 1, dans laquelle la liaison entre la portion sulfoquinovosyle et la portion glycéryle dans la formule (1) est dans la configuration α-anomère.

3. Utilisation selon la revendication 1, dans laquelle la liaison entre la portion sulfoquinovosyle et la portion glycéryle dans la formule (1) est dans la configuration β-anomère.

4. Utilisation selon l'une quelconque des revendications 1 à 3, dans laquelle R représente un groupe alkyle.

5. Utilisation selon la revendication 4, dans laquelle R a 13 à 25 atomes de carbone.

6. Utilisation selon la revendication 5, dans laquelle à la fois R₁₀₁ et R₁₀₂ sont CH₃(CH₂)₁₄CO-.

7. Utilisation selon la revendication 5, dans laquelle à la fois R₁₀₁ et R₁₀₂ sont CH₃(CH₂)₁₆CO-.

8. Utilisation selon l'une quelconque des revendications 1 à 3, dans laquelle R représente un groupe alcényle.

9. Utilisation selon la revendication 8, dans laquelle R a 13 à 25 atomes de carbone.
